# EUROPÄISCHE PATENTSCHRIFT

(11) **EP 0 748 440 B1**
(45) Veröffentlichungstag und Bekanntmachung des Hinweises auf die Patenterteilung: **31.03.1999**
(21) Anmeldenummer: 95942648.7
(22) Anmeldetag: 23.12.1995
(51) Int. Cl.: G01N 3/42, B29C 47/92

(54) **ANORDNUNG ZUR ÜBERWACHUNG UND STEUERUNG DER MATERIALZUSAMMENSETZUNG UND PLASTISCHEN ODER BILDSAMEN VERFORMUNG DES MASSESTROMES EINER MASCHINE**
SYSTEM FOR MONITORING AND CONTROLLING THE MATERIAL COMPOSITION AND PLASTIC OR DUCTILE DEFORMATION OF THE SUBSTANCE FLOW IN A MACHINE
SYSTEME PERMETTANT DE SURVEILLER ET DE REGULER LA COMPOSITION DE MATERIAUX ET D'EFFECTUER UNE DEFORMATION PLASTIQUE OU DUCTILE DU FLUX DE MATIERE PASSANT DANS UNE MACHINE

(30) Priorität: 28.12.1994 DE 4446933
(43) Veröffentlichungstag der Anmeldung: 18.12.1996
(73) Patentinhaber: Innovatherm Prof. Dr. Leisenberg GmbH + Co. KG, D-35510 Butzbach (DE)
(72) Erfinder: RATZENBERGER, Hansgeorg, D-99425 Weimar (DE); GRÖBER, Volker, D-07743 Jena (DE)
(74) Vertreter: Engelhardt, Guido, Dipl.-Ing.
(86) Internationale Anmeldenummer: DE9501866
(87) Internationale Veröffentlichungsnummer: WO9620396

(56) Entgegenhaltungen:
- EP-A- 0 171 911
- DE-C- 522 426
- DE-C- 733 961
- FR-A- 2 558 594
- US-A- 4 097 566
- PATENT ABSTRACTS OF JAPAN vol. 017, no. 455 (M-1466), 20.August 1993 & JP,A,05 104612 (TOYODA GOSEI CO LTD), 27.April 1993,

## Beschreibung

### Technisches Gebiet

Die Erfindung betrifft eine Anordnung zur Überwachung und Steuerung der Materialzusammensetzung und plastischen oder bildsamen Verformung des Massestromes einer Maschine.

Die Erfindung findet dort Anwendung, wo ein plastisch verformbarer bzw. im bildsamem Zustand verformbarer oder verformter Massestrom im Produktionsprozeß auftritt. Das betrifft insbesondere Prozesse in der keramischen Industrie und bei der Herstellung von Lebensmitteln.

### Stand der Technik

Bekannt sind Meßgeräte zur Ermittlung von Materialkonsistenzen, die Penetrometer genannt werden. Der Eindringkörper kann die Form einer Nadel, eines Stabes, eines Kegels, einer Kugel haben oder ähnliche Formen aufweisen. Seine Größe kann zwischen der eines Streichholzes und der eines Bleistiftes liegen. Das Eindringen erfolgt im freien Fall bei vorgewählter Auflast oder unterstützt durch eine Federkraft.
Es wird die Eindringtiefe in das zu untersuchende Material gemessen. Bei Bedarf und bei geeignetem Eindringkörper kann auch der zeitabhängige Verlauf bis zum Erreichen des Eindring-Endes gemessen werden. Die Tiefe der Eindringung wird als Maß für die Konsistenz genutzt. *(Bedienanleitung zu "Automatisches Penetrometer AP 1" Hersteller: VEB Feinmeß Dresden, 1972)* und (U. *Hoffmann und D. Mannheim. "Anwendung des Federpenetrometers 'Solitest"'in "Die Ermittlung von praxisonentierten Kenndaten zur Charakterisierung von Tonen und tonkeramischen Massen" Fortschnttsbenchte der DKG Band 8* (1993) *Heft 1- Charakterisierung toniger Rohstoffe und tonkeramischer Massen -* ).
Mit dem Penetrometer können nur Messungen von Hand oder von Hand ausgelöst durchgeführt werden, die in diskreten Abständen erfolgen.

In *Boström, S.: Kautschuk-Handbuch Berliner Union Stuttgart 1962 S. 131 - 138,* werden verschiedene Geräte zur Messung der Weichheit und Härte beschrieben. Die Weichheitsprüfung hat eine sehr große Ähnlichkeit mit der Härteprüfung nach Brinell, nur daß der Kugeldurchmesser größer (hier 10 mm statt 5 mm) und die Belastung geringer ist (hier 1000 g statt 50 kg). Die Weichheitszahl ergibt sich aus dem Unterschied zwischen den Eindringtiefen einer polierten gehärteten Stahlkugel bei einer Vorlast von 50 g und einer Hauptlast von 1000 g.

In der DE 34 34 904 C1 werden ein Verfahren und eine Anlage zum Überwachen eines aus einer oder mehreren Mischungen bestehenden, kontinuierlich extrudierten Profilbandes oder thermoplastischen Kunststoffes beschrieben.
Die in einer Kühlstrecke auftretenden Längenschrumpfungswerte des Profilbandes werden mechanisch-elektrisch oder optisch-elektronisch erfaßt und die abgetasteten Werte werden an ein mit einer Metergewichtswaage verbundenes Regelgerät gegeben, das den Sollschrumpfungswert einstellt.
In den in der US 4,097,566 und in der US 4,609,336 beschriebenen Lösungen sollen ebenfalls mittels elektronischer und/oder mechanischer Meßwertaufnehmer die Abmessungen eines extrudierten Foliebandes ermittelt und Regelgrößen zur Beeinflussung der Abmessungen des Foliebandes gewonnen werden.
Mit den beschriebenen Geräten ist eine kontinuierliche Messung der Konsistenz oder Weichheit oder Härte nicht möglich. Mit den dort beschriebenen Meßverfahren kann nicht auf die Materialzusammensetzung einer Mischung geschlossen werden.

### Aufgabe der Erfindung

Die Erfindung soll das Problem lösen, Ströme von Medien in Produktionsprozessen fortwährend zu analysieren, um aus den Meßwerten Kenngrößen des Produktionsprozesses und der Materialzusammensetzung zu ermitteln und zu speichern, die der Material analyse und/oder Maschinenüberwachung dienen. Die Kenngrößen sollen auch zur Ermittlung von Steuergrößen für die Beeinflussung des Produktionsprozesses geeignet sein und zur Qualitätsdokumentation im Produktionsprozeß dienen.

### Wesen der Erfindung

Die Aufgabe wird erfindungsgemäß mit einer Anordnung gemäß Anspruch 1 gelöst.
Die Unteransprüche 2 bis 10 sind vorteilhafte Ausgestaltungen des Hauptanspruchs 1.

Die Anordnung zur Überwachung und Steuerung der Materialzusammensetzung und plastischen oder bildsamen Verformung des Massestromes ist an einer Maschine angebracht, die einen abgezweigten Teil eines Massestromes zu einem profilierten Massestrang nur zum Zweck der Messung formt, oder aus dem Gesamtmassestrom einen profilierten Massestrang als Zwischenprodukt erzeugt, an dem gemessen wird. Die Anordnung besteht aus einem an der Maschine drehbar gelagerten Hebel, der mit einer Kraft beaufschlagt ist. Dabei wirkt eine Kraftkomponente im rechten Winkel zur Bewegungsrichtung des profilierten Massestranges oder des Formlings. An dem Hebel ist - an dem in Richtung des profilierten Massestranges oder des Formlings liegenden Hebelende - ein Eindringkörper angebracht, der in Richtung des profilierten Massestranges oder Formlings keilförmig auslaufend ist. Ein erster Wegaufnehmer, zur Ermittlung der Eindringtiefe des Eindringkörpers in den profilierten Massestrang oder Formling korrespondiert in einem Abstand von dem Eindringkörper mit dem Hebel und ist bezüglich der Maschine fest.
Ein zweiter Wegaufnehmer, zur Ermittlung der Vortriebsgeschwindigkeit des profilierten Massestranges, ist ortsfest zur Maschine angeordnet und ist in Kontakt mit dem profilierten Massestrang. Dieser Teil der Anordnung stellt ein kontinuierlich messendes Penetrometer dar.
In einem weiteren Teil der Anordnung weden die Signale, die durch die Auslenkung des ersten Wegaufnehmers und die Signale, die durch den zweiten Wegaufnehmer gewonnen werden, einer Auswerte-, Registrier,- Speicher- und/oder Steuereinheit zugeführt, die mit mindestens einem Stellglied verbunden ist.

Die Auswerte-, Registrier,- Speicher- und/oder Steuereinheit dient der Produktionsüberwachung und Produktionssteuerung.

Hier werden unter dem Begriff "Maschine zur plastischen oder bildsamen Aufbereitung und Verformung eines Massestromes" Einrichtungen verstanden, die in stoffumwandelnden und stofformenden Industriezweigen Verwendung finden, insbesondere in der Lebenmittelindustrie, der chemischen Industrie, der keramischen Industrie und der Baustoffindustrie; dort wo plastische oder bildsame Materialen als Vorprodukte, Zwischenprodukte oder Endprodukte hergestellt werden, das heißt, wo Maschinen, die Komponenten vermischen, Plastizität / Bildsamkeit einstellen, Homogenität herstellen und profilierte Massestränge oder Formlinge formen (pressen), eingesetzt werden.
Die Anordnung kann am Eingang der Maschine, am Ausgang der Maschine oder im Laufe des Maschinenprozesses in der Maschine angeordnet sein.

In einem ersten Fall ist der Eindringkörper ein an dem Hebelende drehbar gelagertes Laufrad, das zum Außendurchmesser keilförmig auslaufend ist, wobei die Radschneide einen Radius von 0,05 mm bis 2 mm hat. Die Auslenkung des Laufrades um die Hebelachse wird mit dem ersten Wegaufnehmer und die Geschwindigkeit des Massestranges wird mit dem zweiten Wegaufnehnmer, der als Drehwinkelgeber ausgebildet ist und mit dem Laufrad korrespondiert, registriert.

In einem zweiten Fall ist der Eindringkörper ein feststehender Gleitkörper, der eine konische Form hat und dessen Schneide einen Radius von 0,05 mm bis 2 mm hat. Zur Messung der Geschwindigkeit des profilierten Massestranges ist der zweite Wegaufnehmer vorgesehen, der als ein an einem weiteren Hebel gelagertes, den profilierten Massestrang berührendes Laufrad ausgebildet ist, das mit einem Drehwinkelgeber korrespondiert.

Mit Hilfe der Anordnung ist die gleichzeitige Erfassung weiterer chemischer und/oder physikalischer Kenngrößen vorgesehen, wie zum Beispiel die elektrische Leitfähigkeit des Massestranges. die Materialtemperatur und die elektrische Leistungsaufnahme der betreffenden Antriebsaggregate, um wichtige, den Produktionsprozeß kennzeichnende Parameter kontinuierlich zu erfassen.

Die Auswerte-, Registrier-. Speicher- und/oder Steuereinheit ist mit mindestens einem Stellglied an der Maschine verbunden, mit dem zumindest eine Komponente der Zusammensetzung des in der Maschine verarbeiteten Massestromes einstellbar ist.

Stellglieder können sowohl vor dem Eingang in die Maschine als auch am Ausgang der Maschine angeordnet sein.

Die Messung kann an dem zum Beispiel verpreßten profilierten Materialstrang (Zwischenprodukt) erfolgen oder aus dem Fertigungsprozeß wird ein Teil-Massestrom abgezweigt und zu einem Meß-Massestrang profiliert, an dem Messungen vornehmbar sind. Das Material des Meß-Massestrangs wird dem Hauptstrom nach der Messung wieder zugeführt.
Die Einstellung / Korrektur der Masse ist vor oder nach der Aufteilung des Massestromes mit Hilfe eines Stellgliedes, zum Beispiel zur Feuchtedosierung, möglich.
Gleichartige Meßgeräte können vor einer Verarbeitungsmaschine (z.B. Strangpresse) zur Überwachung der Zusammensetzung des Vorproduktes (Massestrom) und nach der Verarbeitungsmaschine zur Überwachung der Verarbeitungsmaschine und des Zwischen- oder Endproduktes (Massestrang oder Formling) angeordnet sein. Die gewonnenen Meßwerte werden jeweils einer Auswerte-, Registrier-, Speicher- und/oder Steuereinheit zugeführt.

Mit Hilfe der Erfindung ist es möglich, den Produktionsablauf ständig zu überwachen, auszuwerten, unmittelbar aktiv zu beeinflussen und zu dokumentieren.

Die mit Hilfe der Meßanordnung gewonnenen Meßwerte erhalten eine Kurzzeit- und eine Langzeitinformation.
Die Kurzzeitinformationen geben Informationen über einen momentanen
Grobkornanteil und die momentane Materialfeuchte (als Faktor für die Bildsamkeit). Die Langzeitinformationen dienen der Produktionssteuerung und -überwachung und der Dokumentation. Eine Mittelwertbildung in bestimmten Zeitabschnitten liefert eine Aussage über die Konsistenz des Massestromes (z.B. Feuchtigkeitsgehalt). Die Werte mittlere Amplitude im Zeitintervall und/oder die Periodendauer liefern eine Strang- oder Presseninformation.

### Kurze Beschreibung der Figuren

Die Erfindung wird am Beispiel eines Prozesses in der Tonkeramik-Industrie beschrieben. Es zeigen:
- Figur 1:: Letzte Prozeßstufe zur Herstellung eines schneckenverpreßten profilierten Massestranges,
- Figur 2:: Kontinuierlich registrierendes Penetrometer mit integrierter Vorrichtung zur gleichzeitigen Erfassung der Strangvortriebsgeschwindigkeit,
- Figur 3:: Anordnung zur Überwachung und Steuerung einer Maschine zur plastischen Verformung eines Massestromes,
- Figur 4:: Darstellung von Meßwerten in Abhängigkeit von der Zeit,
- Figur 5:: Prinzipdarstellung zur Erfassung der Meßdaten,
- Figur 6:: Vergrößerte Darstellung der Meßdaten,
- Figur 7:: Schematische Darstellung der Abtastung eines Grobkorn in einem profilierten Massestrang.
- Figur 8:: Kontinuierlich registrierendes Penetrometer mit Vorrichtung zur gleichzeitigen Erfassung der Strangvortriebsgeschwindigkeit und der makroskopischen Strangform

### Wege zur Ausführung der Erfindung

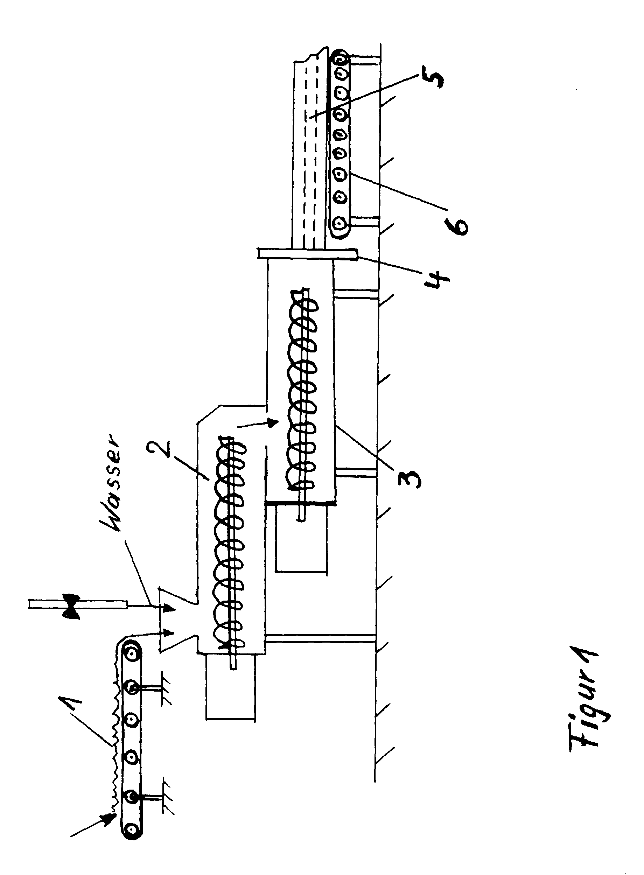

Gemäß der **Figur 1** gelangt ein in Verarbeitung befindlicher Massestrom 1 (z.B. Ton) in ein Aufbereitungsaggregat 2, in dem der Masse bei Bedarf Wasser zugegeben wird und die Masse gut durchmischt wird. Das Aufbereitungsaggregat 2 übergibt die Masse in eine Presse 3, wobei mit Hilfe eines Mundstücks 4 ein profilierter Massestrang 5 erzeugt wird. Der profilierte Massestrang 5 wird mittels eines Transportbandes 6 unterstützt, weitergeleitet und einer Weiterverarbeitung zugeführt.

Die Anordnung dient der Überwachung der momentanen Konsistenz und Zusammensetzung, insbesondere des Grobkorngehaltes und der Verarbeitungsfeuchte des in Verarbeitung befindlichen Massestromes 1 bzw. des Massestranges 5 und/oder des Prozesses der Herstellung des Massestranges 5.

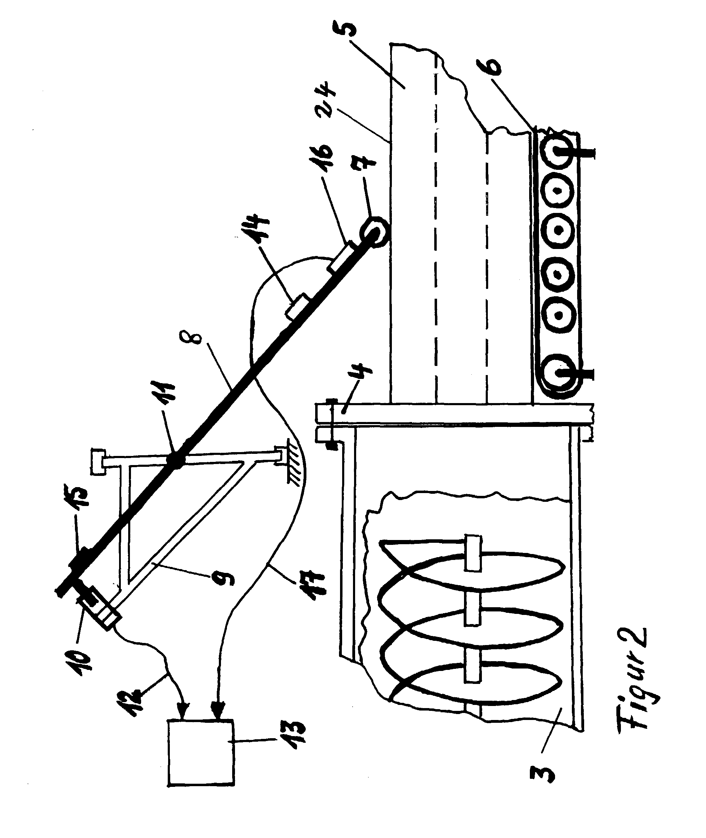

Der Teil der Anordnung , der als kontinuierlich registrierendes Penetrometer bezeichnet wird, besteht gemäß **Figur 2** aus einem über den Massestrang 5 rollenden leichtgängig drehbaren Eindringkörper 7, der im Beispiel als Laufrad ausgebildet ist, das an einem spielfrei gelagerten Hebel 8 angebracht ist. Die Lagerung des Hebels erfolgt mittels eines Halterungssystems 9 im Winkel von etwa 45 ° zur Oberfläche 24 des Massestranges 5. Das Halterungssystem 9 ist in Bezug zum Pressenmundstück 4 ortsfest angeordnet.

Mit dem Hebel 8 korrespondiert in einem Abstand vom Eindringkörper 7 ein Wegaufnehmer 10. Abhängig von der Art des Wegaufnehmers 10 und der zu messenden Eigenschaften des Massestranges 5 kann eine bestimmmte Hebelübersetzung gewählt werden.

Im Beispiel befindet sich der Wegaufnehmer oberhalb des Drehpunktes 11 des Hebels 8. Der Wegaufnehmer 10 ist über eine Meßleitung 12 mit einem geeigneten Meß- und Auswertegerät 13 verbunden.

Die Einstellung der Eindringdruckkraft des Eindringkörpers 7 in den Massestrang 5 erfolgt durch Auflegen von Auflastgewichten 14 und/oder Gegengewichten 15 und wird auf einen konstanten Wert eingestellt. Dabei muß das Schwingungs- und Trägheitsverhalten des Systems Eindringkörper - Hebel - Eindringdruckkraft - Meßwertaufnehmer auf die Meßwerterfassung berücksichtigt werden.

In Abhängigkeit von der Konsistenz des Massestranges 5 und seiner Zusammensetzung dringt der Eindringkörper unterschiedlich tief in den Massestrang ein. Die Änderung der Eindringtiefe wird über die Zeit registriert. In Abhängigkeit von der Geschwindigkeit des Massestranges 5 wird der rotierende Eindringkörper (Laufrad) mit wechselnder Geschwindigkeit bewegt. Da es in der Praxis Schwankungen in der Vortriebsgeschwindigkeit des Massestranges gibt, ist es notwendig, die Vortriebsgeschwindigkeit des Massestranges in die Datenerfassung einzubeziehen. Aus diesem Grund korrespondiert mit dem Eindringkörper 7, der als Laufrad ausgebildet ist, ein Wegaufnehmer 16. Der Wegaufnehmer 16 ist an den Hebel 8 in der Nähe der Lagerung des Laufrades eingebaut und ermittelt die Drehbewegung des Laufrades. Zur Datenerfassung ist der Wegaufnehmer 16 durch eine Meßleitung 17 mit dem Meß- und Auswertegerät 13 verbunden. Ist der Eindringkörper 7 als Laufrad ausgebildet, liegt der Durchmesser bei 5 cm. Die in das Material eindringende Schneide des Laufrades hat einen Radius von 0,1 mm. Die Ausbildung der Schneide wirkt sich auf das Meßverhalten aus. Ein Eindringkörper mit spitzem Konus oder scharfer Gleit- bzw. Lauffläche reagiert bei geringer Auflast sensibler auf Körnungsunterschiede - als Eindringkörper mit großen Abrundungen bei erhöhten Auflasten oder Trägheiten.

Je nach Zusammensetzung des tonkeramischen Rohmaterials bzw. des Massestromes 1 und in erster Näherung abhängig vom momentanen Feuchtegehalt des Massestranges 5 drückt sich der Eindringkörper 7 tiefer oder weniger tief in den Massestrang 5 ein. Der Eindringkörper 7 reagiert aber nicht nur auf die momentane Konsistenz des Massestranges 5, sondern auch auf das in der Masse enthaltene Grobkorn, auf Formgebungseinflüsse und anderes.

Die sich ständig geringfügig ändernde Eindringbewegung wird über den Wegaufnehmer 10 erfaßt. Die Meßwerte des Wegaufnehmers 10 und die Meßwerte des Wegaufnehmers 16 werden mit Hilfe spezieller Hard- und Software in einem Computer des Meß- und Auswertegerätes gespeichert, graphisch dargestellt und mathematisch-statistisch weiterverarbeitet.

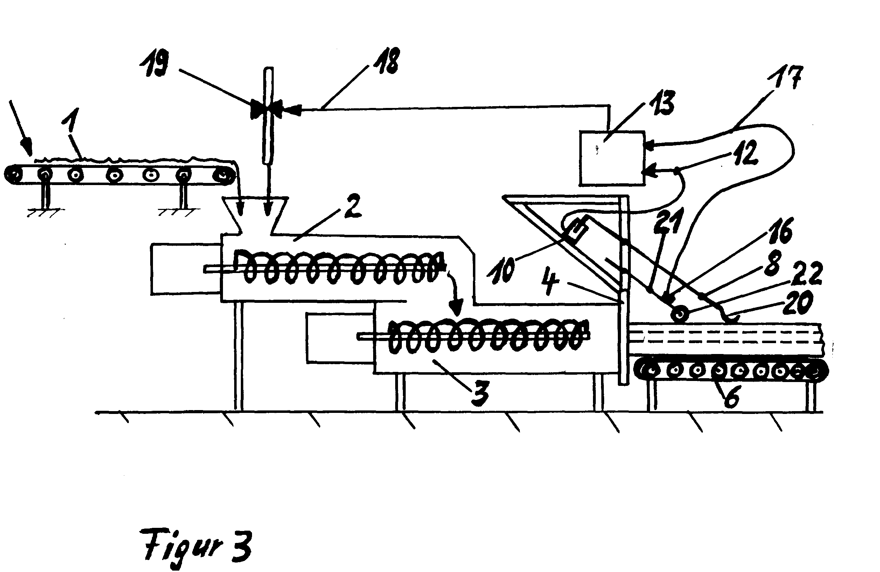

**Figur 3** zeigt eine Anordnung zur Überwachung und Steuerung einer Maschine zur plastischen Verformung eines Massestromes. Bei Erreichen von Grenzwerten, die die Auswerte-, Registrier-, Speicher- und/oder Steuereinheit 13 ermittelt, werden über Steuerleitungen 18 Stellwerte z.B. für eine Wasserdosierung an ein Dosiersystem 19 ausgegeben.
In Figur 3 ist das kontinuierlich messende Penetrometer gegenüber Figur 2 modifiziert aufgebaut. Danach kann der Eindringkörper 7 auch als Gleitkörper 20 ausgebildet sein. In diesem Fall erfolgt die Messung der Strangvortriebsgeschwindigkeit zweckmäßigerweise durch ein eigenständiges System. Dazu ist an einem zweiten Hebel 21 ein zweites Laufrad 22 mit dem Wegaufnehmer 16 so angeordnet, daß ein ständiger gleichmäßiger Kontakt mit der Oberfläche 24 des Massestranges 5 gewährleistet ist.
Mit Hilfe des zweiten Laufrades 22 ist es vorteilhaft, weitere Messungen physikalischer und chemischer Größen, wie zum Beispiel Messung der elektrischen Leitfähigkeit, durchzuführen.

Mit Hilfe der Erfindung ist es möglich, den Produktionsablauf ständig zu überwachen, auszuwerten, unmittelbar aktiv zu beeinflussen und zu dokumentieren. Die mit Hilfe des kontinuierlich registrierenden Penetrometers gewonnenen Meßwerte enhalten eine Kurzzeit- und eine Langzeitinformation.

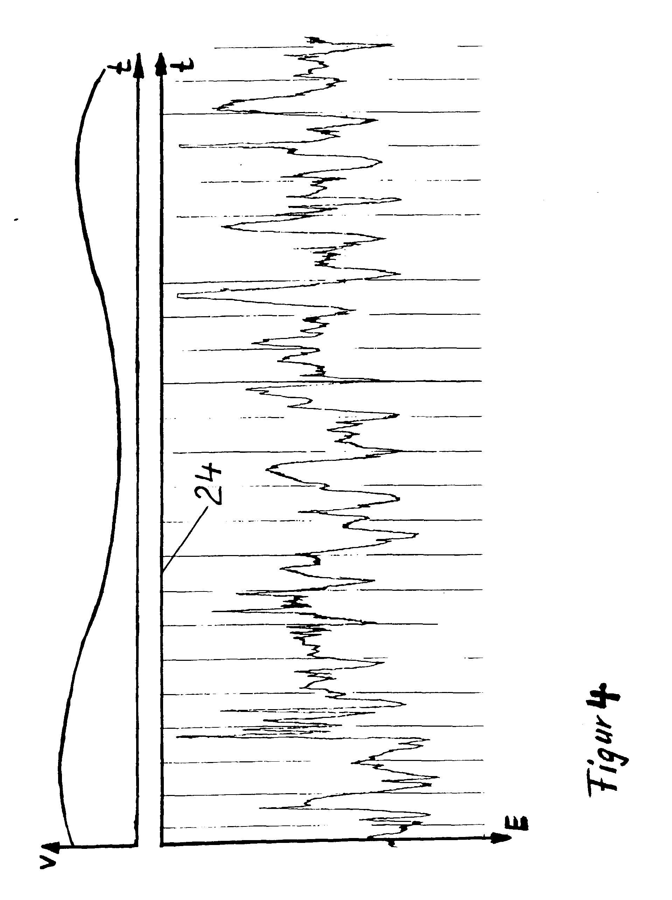

**Figur 4** gibt ein Beispiel für eine graphisch dargestellte Kurzzeitinformation über einen Meßzeitraum von etwa einer Minute, die an einem realen Massestrang ermittelt wurde. Die obere Kurve stellt die Änderung der Geschwindigkeit des Massestranges 5 über die Zeit dar. Die untere Kurve gibt die Änderung der Eindringtiefe E des Eindringkörpers 7 in den Massestrang 5 über die Zeit wieder.

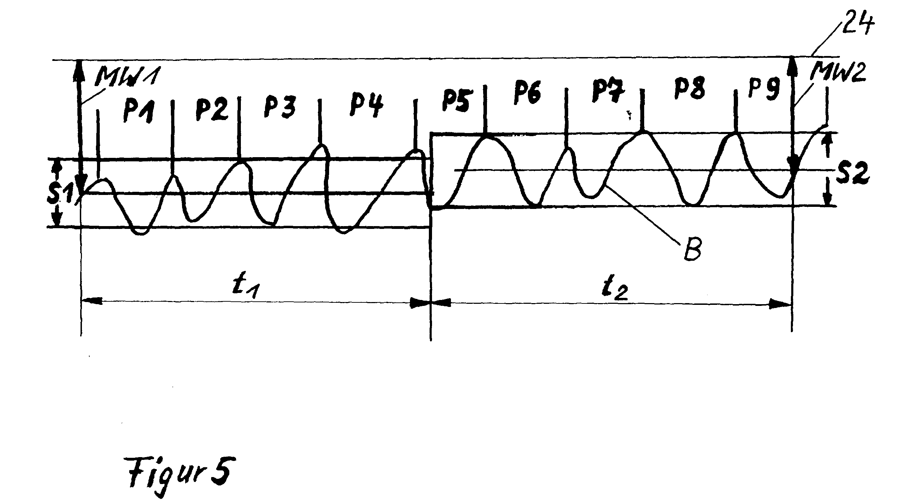

**Figur 5** ist eine schematische Darstellung der Basislinie B der in den Meßdaten enthaltenen Informationen. Unter der Voraussetzung einer konstanten stofflichen Zusammensetzung liefert ein Mittelwert MW₁ der Eindringtiefe über einen Zeitraum t1 von z.B. einer Minute einen Wert für die momentane Konsistenz bzw. den momentanen Feuchtegehalt.

Der Mittelwert MW₁ entspricht dem Sollwert. Bei einem geringeren Feuchtegehalt der Masse tritt in einem folgenden Meßzeitraum t2 eine geringere mittlere Eindringtiefe auf, die zu einem Mittelwert MW₂ führt.
Diese Schwankungen werden nach Intensität und entsprechender Häufigkeit der Zeiträume zur Aktivierung von Stellgliedern zur Feuchtedosierung genutzt. Die Anzahl der Meßzeiträume sollte möglichst groß sein.
Die Perioden P₁ bis P₉ usw. sind spezifische Maschinenkenngrößen und sie werden zur Überwachung der Arbeitsweise der Presse 3 und des Transportbandes 6 ausgewertet. Gleiches gilt auch für die mittlere Amplitudenhöhe S₁ und S₂ usw. im jeweiligen Zeitintervall (Schwankungsbreite).

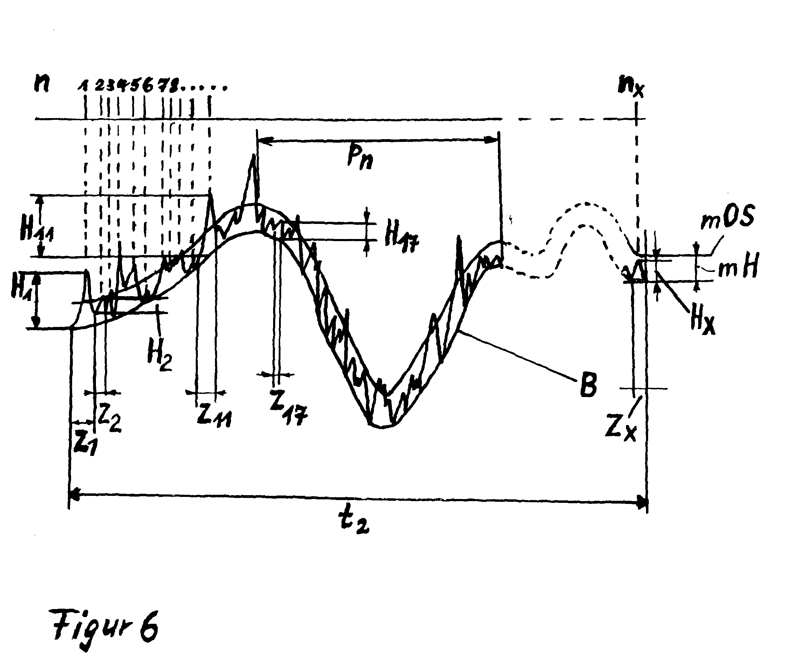

Der in **Figur 6** dargestellte gezoomte Kurvenausschnitt zeigt, daß der Basislinie B der Meßkurve "hochfrequente' Schwingungen in Form von Artefakten oder Transienten überlagert sind. Diese Schwingungen haben ihre Ursache in dem in der Masse enthaltenen Grobkornanteil.
Ihre Frequenz liegt in der Größenordnung zwischen ca. 0,1 und 10 Hertz. Die Größe mH ist die mittlere Amplitudenhöhe der Oberschwingungen, die Größe H ist die Höhe der einzelnen korngrößenbedingten Oberschwingungen, die Größe Z ist die Basisbreite jeder einzelnen grobkornbedingten Oberschwingung, und die Größe n ist die Anzahl der Oberschwingungen pro Zeiteinheit.

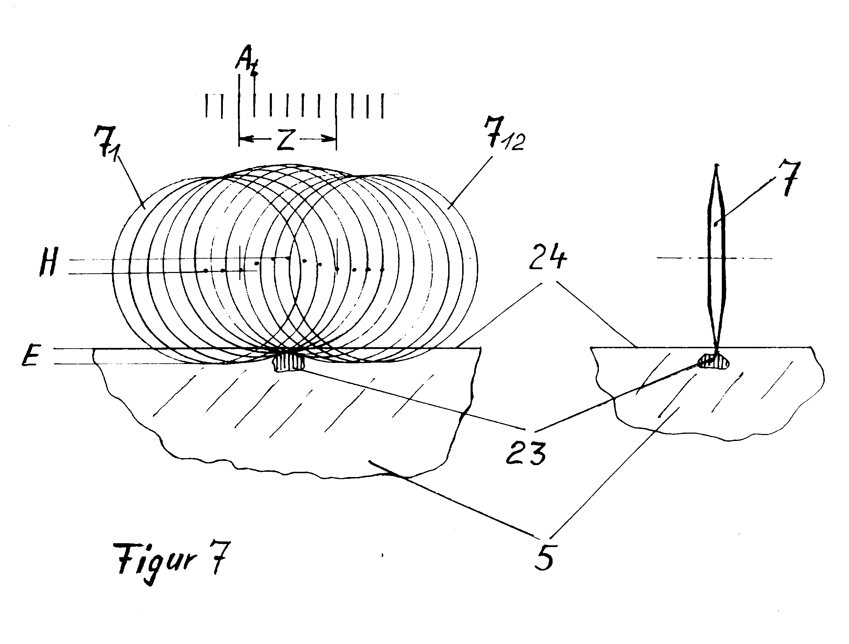

**Figur 7** zeigt, wie ein unter einer Massestrang-Oberfläche 24 befindliches Grobkorn 23 den Eindringkörper 7 bei seiner Eindringung hindert. Die Stellungen 71 bis 712 des Eindringkörpers 7 verdeutlichen, auf welche Weise im Beispiel von Figur 6 eine Amplitudenhöhe H und eine Basisbreite Z entstehen. Aₜ bedeutet die Abtastzeit (Abtastrate), in der auf elektronischem Weg die Änderungen der Eindringtiefe E abgetastet wird.
Die Anzahl dieser Oberschwingungen nₓ pro Zeiteinheit ist ein Maß für den in diesem Zeitraum erfaßbaren Grobkornanteil, ihre mittlere Schwankungsbreite und die mittlere Amplitudenhöhe mH sind ein Maß für die erfaßbare mittlere Partikelgröße. Die Intensität jeder einzelnen Oberschwingung, d.h. die Amplitudenhöhe H jeder einzelnen Schwingung (H₁, H₂, H₃ usw.) und die Basisbreite Z jeder einzelnen Schwingung (Z₁, Z₂, Z₃ usw. ) ist ein Maß für die Größe jedes einzeln erfaßbaren Einzelkorns.

Somit werden aus der Häufigkeit und der Intensität der Oberschwingungen Rückschlüsse auf den Grobkornanteil im Tonstrang gezogen. Mit Hilfe eines speziellen statistischen Computer-Auswerteprogramms werden über den jeweiligen Meßzeitraum der momentane Grobkornanteil und dessen Kornverteilung ermittelt.
Bei konstantem Materialeinsatz ist damit eine Überwachung der Aufbereitungsintensität der betreffenden Aufbereitungsanlage gegeben. Ändert sich dagegen bei konstanten technologischen Bedingungen die Konsistens der Masse (z.B. aufgrund von Schwankungen in der mineralogischen und granulometrischen Zusammensetzung der Einsatzstoffe), so werden diese Schwankungen ebenfalls registriert und können z.B. unter Einbeziehung der elektrischen Leistungsaufnahme der Antriebsaggregate zur automatischen Korrektur der Dosierung der Einsatzstoffe und/oder der Preßfeuchte genutzt werden.
Die Langzeitinformation der Messungen basiert auf der Erfassung und Speichung der in den einzelnen Meßzeiträumen gemittelten Werten der Kurzzeitmessung. Dadurch ergibt sich im Sinn der ISO 9 000 ff. die Möglichkeit zur Darstellung, Speicherung und zum Nachweis von Abläufen z.B. über den Zeitraum einer Arbeitsschicht oder auch eines ganzen Jahreszyklus zwecks Langzeit-Produktionüberwachung und Auswertung.

Werden neben der Messung der Eindringtiefe des kontinuierlich registrierenden penetrometrischen Meßfühlers und der Strangvortriebsgeschwindigkeit auch die Meßwerte der elektrischen Leitfähigkeit und die elektrische Leistungsaufnahme der Antriebsaggregate gleichzeitig dem Computer zugeführt, so können aus den drei Meßgrößen mit Hilfe eines statistischen Rechenprogramms Feuchtigkeitsgehalt, Grobkornanteil, Schwankungen in der Materialzusammensetzung und die Arbeitsweise des Formgebungsaggregates analysiert werden.

Der Grund für die zusätzliche Einbeziehung der elektrischen Leitfähigkeit des Untersuchungsmaterials und der elektrischen Leistungsaufnahme der Antriebsaggregate in die Überwachung liegt darin, daß bei konstanter Massezusammensetzung, konstanter Massefeuchte, konstanter Massetemperatur und konstanten Meßbedingungen auch eine konstante elektrische Leitfähigkeit in einem Material und ein konstanter Elektroenergieverbrauch der Antriebsaggregate vorliegen. Ändert sich die Massezusammensetzung oder auch deren Feuchtegehalt, so ändern sich auch deren elektrische Leitfähigkeit und die elektrische Leistungsaufnahme der Antriebsaggregate. Damit sind die elektrische Leitfähigkeit und die elektrische Leistungsaufnahme der Antriebsaggregate zusätzliche Größen zur Charakterisierung und Überwachung von Masseströmen und Massesträngen.

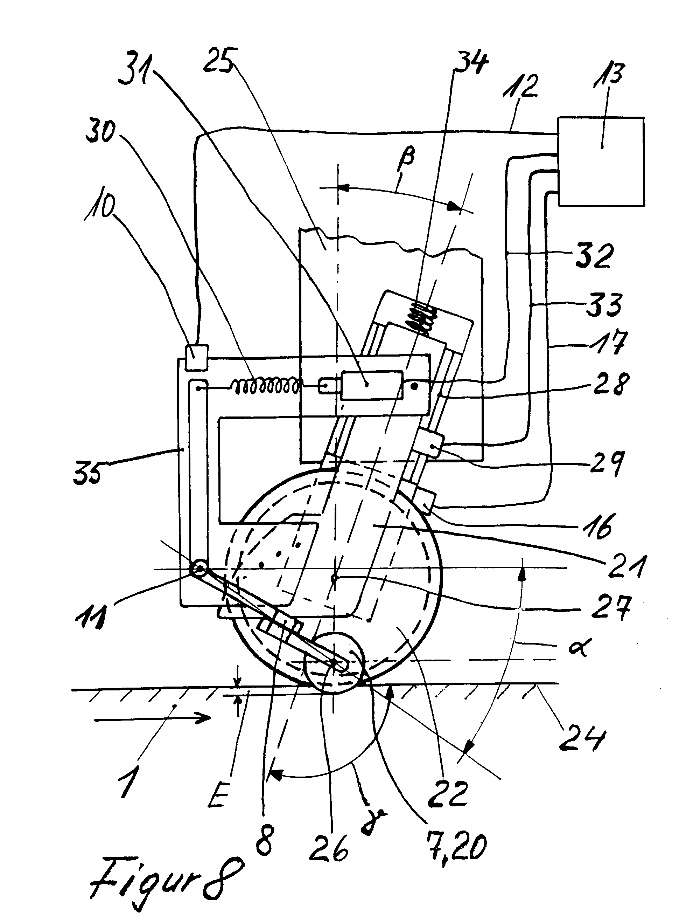

**Figur 8** zeigt ein kontinuierlich registrierendes Penetrometer mit einem hebelgelagerten Eindringkörper 7 in Form eines drehbar gelagerten kegelförmig spitz auslaufenden Abtastrades.

Das Abtastrad ist jedoch auch gegen einen nichtdrehenden Gleitkörper (20) oder eine Abtastnadel austauschbar. Das Abtastrad ist an einem strangseitigen Hebelende in einer ersten Drehachse 26 drehbar gelagert. Der Hebel 8 ist in dem ersten Drehpunkt 11 gelagert, wobei diese Lagerstelle über einen winkelverstellbaren Befestigungswinkel 35 mit dem zweiten Hebel 21 verbunden ist, der in einer Geradführung 28 in einem einstellbaren Winkel β zur Senkrechten auf die Strangoberfläche geführt ist. Der Winkel β kann um bis zu etwa 45° von der Senkrechten abweichen. so daß das Meßsystem gegenüber der Richtung des Preßstranges in einem Winkel γ größer/gleich 90 Grad gegenüberseht. Der Drehpunkt für die Einstellung des Winkels β liegt in der zweiten Drehachse 27.

Der erste Hebel 8 ist in seinem Drehpunkt um etwa 90° bis 140° abgebogen und am strangabsaeitigen Hebelende ist der erste Wegaufnehmer 10 angeordnet, der die Korngrößen und die Konsistens des Massestranges erfaßt.
Dieser Teil des Hebels ist mit einer Zugfeder 30 verbunden, die andererseits mit dem zweiten Hebel 21 verbunden ist. Die Feder 30 hat die Funktion den Eindrinkörper 7 gegenüber dem Massestrang mit einer Kraft zu beaufschlagen. Zur Einstellung der Zugkraft und damit der Druckkraft des Eindringkörpers 7 ist ein Stellmagnet 31 zwischen die Feder 30 und den zweiten Hebel 21 geschaltet, der die Vorspannung der Feder in Stufen einstellt. So sind beispielsweise Messungen bei 80 g oder bei 160 g Gewichtskraft durchführbar.

Der zweite Hebel 21 ist ebenfalls winkelförmig ausgebildet. Das strangabseitige Hebelende ist in der Gleitführung 28 gerade geführt. Die Gleitführung 28 ist mit der Halterung 25 verbunden, die wiederum an der Verarbeitungsmaschine befestigt ist. Im Bereich der Knickstelle des zweiten Hebels 21 ist eine zweite Drehachse 27 für das zweite Laufrad 22 angeordnet. Das zweite Laufrad 22 ermittelt mittels des zweiten Wegaufnehmers 16 die Strangvortriebsgeschwindigkeit. Ein dritter Wegaufnehmer 29 ermittelt die durch das zweite Laufrad übertragenen Bewegungen am zweiten Hebel 21, die von der makroskopischen Form des Massestranges hervorgerufen werden.

Der zweite Hebel 21 hat eine Verstellmöglichkeit für den Befestigungswinkel 35, der den Drehpunkt 11 trägt. Der Drehpunkt 11 ist mit Hilfe dieser Verstellmöglichkeit um die erste Drehachse 26 verstellbar. Damit wird der Eindringwinkel α eingestellt, wobei die erste Drehachse 26 und die zweite Drehachse 27 nahezu (grob angenähert) in einem Lot zur Strangoberfläche sind.

Der Abstand der ersten Drehachse 26 von dem zweiten Drehpunkt 11 liegt im Bereich von einigen Zentimetern und ist so bemessen, daß das angenäherte Lot durch die zweite Drehachse 27 und die erste Drehachse 26 - in Strangvortriebsrichtung gesehen - nach derjenigen Geraden liegt, die der gedachten Linie der Bewegung der Geradführung durch die zweite Drehachse 27 entspricht.

Weiterhin ist zwischen dem Gehäuse 25 und dem zweiten Hebel 21 eine Andruckregulierungsfeder 34 vorgesehen, die es ermöglicht, das Auflagegewicht des zweiten Laufrades 22 einzustellen und weiterhin ermöglicht, die Anordnung seitlich oder von unten (gegen die Richtung der Gewichtskraft) anzuordnen.

Der erste Wegaufnehmer 10 ist über eine erste Meßleitung 12, der zweite Wegaufnehmer 16 ist über eine zweite Meßleitung 17, der Stellmagnet über eine Magnetsteuerleitung 32 und der dritte Wegaufnehmer ist über eine dritte Meßleitung mit der Auswerte- Registrier- Speicher- und/oder Steuereinheit 13 verbunden.

Der Eindringörper 7 und das Laufrad 22 sind mit einer elektrischen Lösespannung zur Reinigung beaufschlagt. Vorzugsweise sind der Eindringkörper 7 und das zweite Laufrad 22 elektrisch voneinander isoliert. Dann sind zwischen dem Eindrinkörper 7 und/oder dem zweiten Laufrad 22 und/oder der Presse Leitfähigkeitsmessungen durchführbar.

### Bezugszeichen

- 1: Massestrom
- 2: Aufbereitungsaggregat
- 3: Presse
- 4: Mundstück
- 5: profilierter Massestrang
- 6: Transportband
- 7: Eindringkörper
- 8: Hebel
- 9: Halterungssystem
- 10: erster Wegaufnehmer
- 11: Drehpunkt
- 12: erste Meßleitung
- 13: Auswerte-, Registrier,- Speicher- und/oder Steuereinheit
- 14: Auflastgewicht
- 15: Gegengewicht
- 16: zweiter Wegaufnehmer
- 17: zweite Meßleitung
- 18: Steuerleitung
- 19: Dosiersystem
- 20: Gleitkörper
- 21: zweiter Hebel
- 22: zweites Laufrad
- 23: Grobkorn
- 24: Oberfläche des Massestranges
- 25: Gehäuse
- 26: erste Drehachse
- 27: zweite Drehachse
- 28: Gleitführung
- 29: dritter Wegaufnehmer
- 30: Feder
- 31: Stellmagnet
- 32: Magnetsteuerleitung
- 33: dritte Meßleitung
- 34: Andruckregulierungsfeder
- 35: Befestigungswinkel
- tᵢ: Zeitintervall
- Si: mittlere Amplitude im Zeitintervall
- t: Zeit
- E: Eindringtiefe
- v: Strangvortriebsgeschwindigkeit
- MWᵢ: Mittelwerte der Eindringtiefe über die Zeitintervalle
- P₁, P₂, ....: Perioden
- B: Basislinie der Meßkurve
- n: Anzahl der Oberschwingungen
- mH: mittlere Schwingungsbreite der korngrößenbedingten mittleren Amplitudenhöhe (Oberschwingung)
- H₁, H₂, ....: Amplitudenhöhe der einzelnen Oberschwingung
- Z₁, Z₂, ...: Basisbreite der einzelnen Oberschwingung
- Aₜ: elektronische Abtastzeit (Abtastrate)

## Patentansprüche

1. Anordnung zur Überwachung und Steuerung der Materialzusammensetzung und plastischen oder bildsamen Verformung des Massestromes einer Maschine, bestehend aus den Massestrom berührenden Meßwertaufnehmern (Wegaufnehmer 10, 16), deren Signale einer Auswerte-, Registrier-, Speicher- und/oder Steuereinheit (13) zuführbar sind, die mit mindestens einem Stellglied (Dosiersystem 19) verbunden ist, mit dem die Zusammensetzung des Massestromes einstellbar ist und weiter bestehend aus:
- einen an der Maschine (4, 3) angebrachten drehbar gelagerten Hebel (8), der mit einer Kraft (14, 15) beaufschlagt ist, wobei eine Kraftkomponente im rechten Winkel zur Oberfläche (24) des Massestranges (5) wirkt,
- einen Eindringkörper (7), der an dem in Richtung des Massestranges (5) liegenden Hebelende angeordnet ist und in Richtung der Oberfläche (24) des Massestranges (5) keilförmig auslaufend ist,
- einen ersten Wegaufnehmer (10), zur Ermittlung der Eindringtiefe (E), der mit dem Hebel (8) in einem Abstand von dem Eindringkörper (7) korrespondiert und der mit der Maschine fest verbunden ist, und
- einen zweiten Wegaufnehmer (16), zur Ermittlung der Vortriebsgeschwindigkeit des Massestranges, der in Richtung des bewegten Massestranges (5) und nahezu ortsfest zur Maschine angeordnet ist.

2. Anordnung nach Anspruch 1, bei der der Eindringkörper (7) ein an dem Hebelende drehbar gelagertes Laufrad ist, das zum Außendurchmesser hin keilförmig auslaufend ist, wobei die Radschneide einen Radius von 0.01 mm bis 2 mm hat und der Außendurchmesser zwischen 1 cm und 10 cm ist.

3. Anordnung nach Anspruch 1, bei der die Auslenkung des Eindringkörpers (7) um die Hebelachse mit dem ersten Wegaufnehmer (10) und die Geschwindigkeit des Massestranges mit dem zweiten Wegaufnehmer (16), der als Drehwinkelgeber ausgebildet ist und mit einem Laufrad korrespondiert, registrierbar sind.

4. Anordnung nach Anspruch 1, bei der der Eindringkörper (7) ein feststehender Gleitkörper ist, der eine zur Oberfläche (24) des Massestranges (5) auslaufende konische Form hat und dessen Schneide einen Radius von 0,01 mm bis 2 mm hat, und der zur Messung der Geschwindigkeit des Massestranges (5) angeordnete zweite Wegaufnehmer als ein an einem zweiten Hebel (21) gelagertes, die Oberfläche (24) des Massestranges (5) berührendes Laufrad (22) mit dem korrespondierenden Drehwinkelgeber (16) ausgebildet ist.

5. Anordnung nach Anspruch 1, bei der eine gleichzeitige Messung weiterer chemischer und/oder physikalischer Kenngrößen, wie elektrische Leitfähigkeit, Materialtemperatur, durchführbar ist.

6. Anordnung nach Anspruch 1, bei der aus dem Fertigungsprozeß ein Meß-Massestrang abzweigbar ist, an dem Messungen vornehmbar sind, und bei der der Meß-Massestrang dem Hauptstom wieder zuführbar und nachfolgend die Einstellung der in Verarbeitung befindlichen Masse durchführbar ist.

7. Anordnung nach Anspruch 1, bei der das zweite Laufrad (22) an einer im Winkel (β) ausgerichteten Geradführung (28) schwenkbar gelagter ist und der Eindringkörper (7) an einem strangseitigen Hebelende des Hebels (8) befestigt ist und der Drehpunkt 11 des Hebels (8) auf einem Befestigungswinkel (35) angeordnet ist, der Befestigungswinkel (35) gegenüber dem zweiten Hebel (21) verstellbar ist und damit ein Winkel (a) einstellbar ist und die Länge der Hebel und ihre Zuordnung so gewählt sind, daß nahezu ein Lot durch die erste Drehachse 27 und den Berührungspunkt des Eindringkörpers (7) mit der Oberfläche des Massestrangs (24) fällbar ist.

8. Anordnung nach Anspruch 1, bei der der Eindringörper (7) und das Laufrad (22) mit einer elektrischen Lösespannung zur Reinigung beaufschlagt sind.

9. Anordnung nach Anspruch 5, bei der der Eindringkörper (7) und das zweite Laufrad (22) und die Presse (3) elektrisch voneinander isoliert sind und zwischen diesen Bauteilen Leitfähigkeitsmessungen durchführbar sind.

10. Anordnung nach Anspruch 1, bei der gleichartige Meßgeräte vor einer Verarbeitungsmaschine zur Überwachung der Zusammensetzung des Vorproduktes und nach der Verarbeitungsmaschine zur Überwachung der Verarbeitungsmaschine und des Zwischen- oder Endproduktes angeordnet sind.

## Claims

1. System for monitoring and controlling the material composition and plastic or ductile deformation of the substance flow in a machine, comprising measuring sensors (position encoders 10, 16) touching the substance flow, the signals from which can be transmitted to an evaluation, registration, memory and/or control unit (13) which is connected to at least one actuator (metering system 19) by means of which the composition of the substance flow can be adjusted, and further consisting of:
- a lever (8) attached to the machine (4, 3) in a rotating mounting to which a force (14, 15) is applied, with a force vector acting at right angles to the surface (24) of the substance billet (5),
- a penetration body (7) arranged on the end of the lever, arranged in the direction of the substance billet (5) and with a wedge-shaped profile running towards the surface (24) of the substance billet (5),
- first position encoder (10) for determining the penetration depth (E), which corresponds to the lever (8) at a distance from the penetration body (7) and which is firmly connected to the machine, and
- second position encoder (16) for determining the speed of propulsion of the substance billet, which is arranged in the direction of the moving substance billet (5) and occupies a practically fixed position relative to the machine.

2. A system in accordance with Claim 1, in which the penetration body (7) is a rotating trolley wheel mounted on the end of the lever, being a wheel with a wedge-shaped profile towards its external diameter and forming a knife wheel with a radius of 0.01 mm to 2 mm and an external diameter of between 1 cm and 10 cm.

3. System in accordance with Claim 1, in which the deflection of the penetration body (7) about the lever axis can be registered with the first position encoder (10) and the speed of the substance billet can be registered with the second position encoder (16) which is configured as a rotary angle encoder and corresponds to a trolley wheel.

4. System in accordance with Claim 1, in which the penetration body (7) is a fixed plain body tapering with a conical profile towards the surface (24) of the substance billet (5), the blade of which has a radius of 0.01 mm to 2 mm, and with the second position encoder for measuring the speed of the substance billet (5) being arranged as a trolley wheel (22) mounted on a second lever (21) and touching the surface (24) of the substance billet (5), together with the corresponding rotary angle encoder (16).

5. System in accordance with Claim 1, in which it is possible to measure further chemical and/or physical parameters at the same time, such as electrical conductivity, material temperature.

6. System in accordance with Claim 1, in which it is possible to branch off a measurement substance billet from the production flow for performing measurements on it, and in which the measurement substance billet can be returned to the main flow, after which the substance being processed at the time can be adjusted.

7. System in accordance with Claim 1, in which the second trolley wheel (22) is in a rotating mounting on a straight guide (28) arranged at an angle (β), and the penetration body (7) is fixed onto an end of the lever (8) at the billet end and the fulcrum 11 of the lever (8) is arranged on a fixing bracket (35), with the fixing bracket (35) being adjustable in relation to the second lever (21), thereby allowing an angle (α) to be set, and the length and allocation of the lever (21) are selected so that it is almost possible to sink a plumb line through the first axis of rotation 27 and the point at which the penetration body (7) contacts the surface of the substance billet (24).

8. System in accordance with Claim 1, in which the penetration body (7) and the trolley wheel (22) carry an electrical repelling voltage for cleaning purposes.

9. System in accordance with Claim 5, in which the penetration body (7) and the second trolley wheel (22) and the press (3) are electrically isolated from one another, with it being possible to perform conductivity measurements between these components.

10. System in accordance with Claim 1, in which the same types of measuring instrument are arranged prior to a processing machine for monitoring the composition of the preliminary product and after the processing machine for monitoring the processing machine and the intermediate or finished product.

## Revendications

1. Système permettant de surveiller et de réguler la composition de matériaux et d'effectuer une déformation plastique ou ductile du flux de matière passant dans une machine, comprenant des capteurs de mesure en contact avec le flux de matière (capteurs de course 10, 16), dont les signaux se laissent diriger vers une unité d'évaluation, d'enregistrement, de mémorisation et/ou de régulation (13) raccordée avec au moins un actuateur (système de dosage 19) permettant la régulation de la composition du flux de matière, et comprenant de plus
- un levier rotatif (8) disposé sur la machine (4, 3) qui est soumis à une force (14, 15), une composante de force agissant perpendiculairement sur la surface (24) de la matière (5),
- un corps pénétrant (7) disposé sur l'extrémité de levier orientée en direction de la matière (5) et ayant la forme d'un coin dirigé vers la surface (24) de la matière (5),
- un premier capteur de course (10) pour déterminer la profondeur de pénétration (E) collaborant avec le levier (8) à une certaine distance du corps pénétrant (7) et solidaire de la machine, et
- un deuxième capteur de course (16) pour déterminer la vitesse d'avance de la matière, disposé en direction de la matière déplacée (5) et quasiment stationnaire par rapport à la machine.

2. Système d'après la revendication 1, avec comme corps pénétrant (7) une roue de roulement tournant sur l'extrémité du levier et ayant, vers le diamètre extérieur, la forme d'un coin, la coupe de la roue ayant un rayon de 0,01 à 2 mm et le diamètre extérieur étant d'entre 1 et 10 cm.

3. Système d'après la revendication 1, la déviation du corps pénétrant (7) autour de l'axe du levier se laissant enregistrer au moyen du premier capteur de course (10) et la vitesse de la matière avec le deuxième capteur de course (16) conçu sous la forme d'un transmetteur d'angle et collaborant avec une roue de roulement.

4. Système d'après la revendication 1, avec comme corps pénétrant (7) un corps lisse stationnaire ayant la forme d'un coin dirigé vers la surface (24) de la matière (5) et dont la coupe a un rayon de 0,01 à 2 mm, et dont le deuxième capteur de course prévu pour mesurer la vitesse de la matière (5) est conçu sous la forme d'une roue de roulement (22) logée sur un deuxième levier (21) et touchant la surface (24) de la matière (5) avec le transmetteur d'angle (16) correspondant.

5. Système d'après la revendication 1, permettant simultanément la mesure d'autres paramètres chimiques et/ou physiques tel que la conductance électrique, la température du matériel etc.

6. Système d'après la revendication 1 dans le processus de fabrication duquel il se laisse prélever un échantillon de mesure, dans lequel cet échantillon se laisse ensuite ajouter à nouveau au flux de matière principal et qui permet ensuite de réguler la masse subissant le traitement.

7. Système d'après la revendication 1, dans lequel la deuxième roue de roulement (22) est disposée de manière pivotante sur un guidage droit (28) orienté sous un angle (β), dans lequel le corps pénétrant (7) est fixé sur une extrémité du levier (8) côté matière, le point d'appui 11 du levier (8) se trouvant sur un angle de fixation (35), l'angle de fixation (35) se laissant régler par rapport au deuxième levier (21), de sorte qu'il se laisse régler un angle a et que la longueur des leviers et leur assignation soient choisies tel qu'il se laisse abaisser une perpendiculaire à peu près à travers le premier axe de rotation 27 et le point de contact du corps pénétrant (7) sur la surface de la matière (24).

8. Système d'après la revendication 1, dans lequel le corps pénétrant (7) et la deuxième roue de roulement (22) sont soumis à une tension électrique de détachement pour le nettoyage.

9. Système d'après la revendication 5, dans lequel le corps pénétrant (7), la deuxième roue de roulement (22) et la presse (3) sont électrique-ment isolés l'un de l'autre et dans lequel il est possible de procéder à la mesure de la conductance entre ces éléments.

10. Système d'après la revendication 1, dans lequel il est prévu des unités de mesure similaires, une fois en amont de la machine de traitement pour surveiller la composition de l'avant-produit, d'autre part en aval de la machine de traitement pour surveiller aussi bien la machine que le produit intermédiaire ou le produit fini.
